Europäisches Patentamt

European Patent Office

Office européen des brevets

(11) Veröffentlichungsnummer: **0 157 909**
**B1**

(12) **EUROPÄISCHE PATENTSCHRIFT**

(45) Veröffentlichungstag der Patentschrift:
**08.02.89**

(21) Anmeldenummer: **84113793.8**

(22) Anmeldetag: **10.03.82**

(60) Veröffentlichungsnummer der früheren Anmeldung
nach Art. 76 EPÜ: **0074368**

(51) Int. Cl.⁴: **A 61 K  9/22,** A 61 L  17/00,
A 61 M  31/00

(54) Pharmakahaltige Körperchen.

(30) Priorität: **19.03.81  DE 3110805**
**14.07.81  DE 3127696**

(43) Veröffentlichungstag der Anmeldung:
**16.10.85 Patentblatt 85/42**

(45) Bekanntmachung des Hinweises auf die Patenterteilung:
**08.02.89 Patentblatt 89/6**

(84) Benannte Vertragsstaaten:
**AT CH DE FR GB LI NL SE**

(56) Entgegenhaltungen:
**DE-B- 2 320 373**
**US-A- 3 444 858**

(73) Patentinhaber: **MERCK PATENT GESELLSCHAFT MIT
BESCHRÄNKTER HAFTUNG, Frankfurter
Strasse 250 Postfach 4119, D-6100 Darmstadt (DE)**

(72) Erfinder: **Härle, Anton, Dr., Drechsler Weg 40,
D-4400 Münster-Roxel (DE)**

ACTORUM AG

## Beschreibung

Die Anmeldung betrifft pharmakahaltige Körperchen, bestehend aus einem biologisch inerten Kunststoff, wobei ein oder verschiedene Pharmaka in gleichförmiger Verteilung freisetzbar in dem Kunststoff vorliegen und die Körperchen miteinander verbunden sind.

Aus der DE-OS 23 20 373 ist ein antibiotikahaltiges Mittel in Kugelform bekannt, wobei die Kugeln mit Hilfe von Fäden oder Drähten miteinander verbunden sind. Derartige antibiotikahaltige Hilfsmittel werden in weitem Umfang in der Knochenchirurgie eingesetzt, insbesondere zur Behandlung von posttraumatischer Osteomyelitis. Die miteinander durch einen Faden oder einen dünnen Draht verbundenen Kugeln werden dabei in osteomyelitische Höhlen eingelegt.

Die Kugelform weist eine relativ geringe Oberfläche auf, so dass die nur an der Oberfläche erfolgende Freisetzung des Pharmakon relativ gering ist. Auch hat sich die Kugelform nicht als optimal erwiesen in Bezug auf die leichte Entfernbarkeit einer bereits von Körpergewebe umschlossenen Kette.

Aus der DE-PS 28 06 609 ist es bekannt, Osteosynthesehilfsmittel mit zur Aussenseite hin offenen Aufnahmeräumen für die Aufnahme und Halterung einer Trägermasse zu versehen, wobei in die Trägermasse Antitiobika eingefüllt sind. Gemäss diesem Vorschlag sind in besonderer Weise ausgebildete Osteosynthesehilfsmittel erforderlich oder bei Verwendung bekannter Osteosynthesehilfsmittel müssen die vorhandenen Aufnahmeräume vor der Operation mit dem pastösen antibiotikahaltigen Polymethacrylat oder Polyacrylat gefüllt werden, wobei bis zum Einsatz dieses Hilfsmittels dann die Zeitspanne abgewartet werden muss, in der das Polymethacrylat oder Polyacrylat aushärtet. Die Dosiskontrolle bei einer derartigen Verfahrensweise ist schwierig.

Auch diese Handhabung wurde als zu zeitaufwendig empfunden und durch die Sonderbauteile wurde der Einsatz des neuen Hilfsmittels erheblich erschwert.

Der Erfindung liegt die Aufgabe zugrunde, das an sich aus der DE-PS 23 20 373 bekannte pharmakahaltige Körperchen so in seiner Gestaltung zu verbessern, dass eine leichte Handhabbarkeit und eine leichte, sichere und vollständige Wiederentfernbarkeit aus dem Körpergewebe, Körperhöhlen, Wunden od.dgl. erreicht wird.

Diese der Erfindung zugrundeliegende Aufgabe wird durch die in den Ansprüchen genannten Merkmale gelöst.

Die Körperchen sind durch eingearbeitete Fäden oder Drähte miteinander verbunden, wodurch erreicht wird, dass sie auch dann, wenn bereits Bindegewebe in den Zwischenräumen gewachsen ist, problemlos aus der Wunde entfernt werden können, da die Körperchen durch ihre von der Kugelform abweichende Form einen geringeren Widerstand zu überwinden haben und durch die eingearbeiteten Fäden oder Drähte die Körperchen so fest miteinander verbunden sind, dass ein Abreissen der Körperchen in der Körperhöhle oder in der Knochenhöhle nicht mehr eintreten kann.

Durch die erfindungsgemässe Ausgestaltung, nämlich dadurch, dass die Körperchen wenigstens an einem Ende eine sich verjüngende Form aufweisen, wird ein besseres Gleiten im Körpergewebe im Vergleich zu den bisher bekannten Kugeln sichergestellt, so dass dadurch die Entfernung des Körperchens aus dem Gewebe erleichtert und sichergestellt wird, so dass ein vollständiges Entfernen der Körperchen möglich ist, ohne dass im Gewebe Partikelchen zurückbleiben.

Eine Einscheidung der pharmakahaltigen Körperchen durch dicke und straffe Bindegewebskapseln ist ausgeschlossen. Es entfällt damit die sonst zusätzlich erforderliche, präparatorische Entfernung, die bei den bisher bekannten, frei im Gewebe liegenden Ketten auch bei sorgfältigster, chirurgischer Technik oft nicht vollständig gelingt.

Die kugelelliptische Form schafft weiterhin eine grosse Oberfläche für die Freigabe des Pharmakon.

Ausführungsbeispiele der Erfindung werden nachfolgend anhand der Zeichnung erläutert. Die Zeichnungen zeigen dabei in

Fig. 1 eine Ansicht miteinander druck- und zugfest verbundener elliptischer Körperchen

Fig. 2 und 3 Körperchen, die durch ihre Formgestaltung die Entnahme aus dem Gewebe erleichtern und die vorzugsweise unmittelbar in Wundöffnungen, Knochenhöhlen, Wunden od.dgl. untergebracht werden.

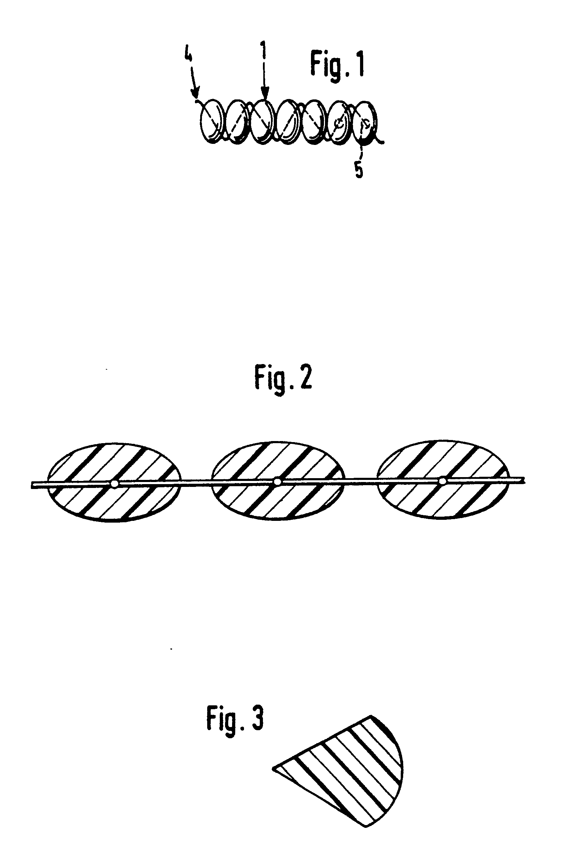

Bei dem in Fig. 1 dargestellten Ausführungsbeispiel sind elliptische Körperchen durch ein zusätzliches Verbindungsmittel 4 miteinander verbunden, wobei die Körperchen selbst aber wiederum materialschlüssig oder durch Verkleben miteinander verbunden sind. Hierbei kann vorzugsweise so vorgegangen werden, dass das Verbindungsmittel 4 im Endbereich der vorgesehenen Körperchenkette mit zusätzlichen Halterungsmitteln 5 ausgerüstet ist, so dass bei einem Zug an der Gesamtkette und bei einem Lösen der Körperchen voneinander die ersten und letzten Körperchen auf jeden Fall fest an dem Verbindungsmittel gehalten werden.

Die Erfindung ist selbstverständlich nicht auf die dargestellten Ausführungsbeispiele beschränkt, sondern es sind demgegenüber sowohl in der Form der Körperchen wie auch in der Art der Verbindung der Körperchen untereinander Abänderungen möglich, ohne dass dadurch der Rahmen der Erfindung verlassen wird.

Bei dem in Fig. 2 dargestellten Ausführungsbeispiel weist das Körperchen die Form eines Rotationsellipsoides auf. Hierdurch werden spitz zulaufende Endflächen geschaffen, die die Entfernung des Körperchens durch Herausziehen od.dgl. erleichtern und ermöglichen.

Die Körperchen sind dabei über Drähte oder Fäden miteinander verbunden, in die zusätzliche Halterungsmittel eingearbeitet sein können. Die Oberfläche wird gegenüber der Kugelform erheblich vergrössert.

Bei dem in Fig. 3 dargestellten Ausführungsbeispiel ist das Körperchen ausgehend von einer Kugel zu einem Kegel umgestaltet, der einerseits eine grosse Freigabefläche besitzt, andererseits aufgrund seiner spitzen Form die Wiederentfernbarkeit erleichtert.

Es wird an dieser Stelle noch einmal ausdrücklich darauf hingewiesen, dass durch die Zeichnungen eine Beschränkung der möglichen Formen nicht bedingt wird.

Die erfindungsgemässen Körperchen geben die Kombinationsmöglichkeit einerseits von verschiedenen Pharmaka, andererseits als Träger für andere resorbierbare Stoffe, wobei in den Ansprüchen 7 bis 11 verschiedene Kombinationsmöglichkeiten erläutert werden.

**Patentansprüche**

1. Pharmakahaltige Körperchen, bestehend aus einem biologisch inerten Kunststoff, wobei ein oder verschiedene Pharmaka in gleichförmiger Verteilung freisetzbar in dem Kunststoff vorliegen und die Körperchen miteinander verbunden sind, dadurch gekennzeichnet, dass die Körperchen eine von der reinen Kugelform abweichende Formgestaltung aufweisen, in die Körperchen Fäden oder Drähte zusätzlich eingearbeitet sind und wenigstens an einem Ende eine sich verjüngende Formgestaltung aufweisen.

2. Pharmakahaltige Körperchen nach Anspruch 1, dadurch gekennzeichnet, dass die Fäden oder Drähte, die in die Körperchen eingearbeitet sind, mit zusätzlichen Halterungsmitteln versehen sind.

3. Pharmakahaltige Körperchen nach Anspruch 1 oder 2, dadurch gekennzeichnet, dass die Körperchen polygonal ausgebildet sind.

4. Pharmakahaltige Körperchen nach einem der Ansprüche 1-3, dadurch gekennzeichnet, dass die Körperchen einen zur festen, aber wieder lösbaren Verankerung in einem Osteosynthese-Hilfsmittel oder in einem Implantat dienenden polygonalen oder gerundeten Querschnitt aufweisen.

5. Pharmakahaltige Körperchen nach einem der Ansprüche 1-4, dadurch gekennzeichnet, dass die Körperchen als Rotationsellipsoid ausgebildet sind.

6. Pharmakahaltige Körperchen nach einem der Ansprüche 1-5, dadurch gekennzeichnet, dass es sich bei dem Pharmakon um antibakteriell wirksame Substanzen handelt.

7. Pharmakahaltige Körperchen nach Anspruch 6, dadurch gekennzeichnet, dass es sich bei dem Pharmakon um Substanzen aus der Gruppe der Antibiotica handelt.

8. Pharmakahaltige Körperchen nach einem der Ansprüche 1-5, dadurch gekennzeichnet, dass es sich bei dem Pharmakon um antiviral wirksame Substanzen handelt.

9. Pharmakahaltige Körperchen nach einem der Ansprüche 1-5, dadurch gekennzeichnet, dass es sich bei dem Pharmakon um Substanzen handelt, die gegen Gewebegeschwülste wirksam sind.

10. Pharmakahaltige Körperchen nach einem der Ansprüche 1-9, dadurch gekennzeichnet, dass Pharmaka mit unterschiedlichen Wirkungen in Kombinationen zur Anwendung kommen.

**Revendications**

1. Corpuscules renfermant des produits pharmaceutiques constitués d'une matière synthétique biologiquement inerte, où un ou différents produits pharmaceutiques se trouvent dans la matière synthétique de manière à pouvoir être libérés avec une répartition uniforme et où les corpuscules sont reliés les unes aux autres, caractérisés en ce que les corpuscules présentent une forme s'écartant de la forme sphérique pure, en ce que des fils métalliques ou autres sont en outre insérés dans les corpuscules et en ce que ceux-ci présentent au moins à leur extrémité une configuration qui se rétrécit.

2. Corpuscules renfermant des produits pharmaceutiques selon la revendication 1, caractérisés en ce que les fils métalliques ou autres qui sont insérés dans les corpuscules sont munis de moyens de fixation supplémentaires.

3. Corpuscules renfermant des produits pharmaceutiques selon la revendication 1 ou 2, caractérisés en ce que les corpuscules ont une forme polygonale.

4. Corpuscules renfermant des produits pharmaceutiques selon l'une des revendications 1-3, caractérisés en ce que les corpuscules présentent une section polygonale ou arrondie servant à un ancrage solide mais à niveau amovible dans un auxiliaire d'ostéosynthèse ou dans un implant.

5. Corpuscules renfermant des produits pharmaceutiques selon l'une des revendications 1-4, caractérisés en ce que les corpuscules ont une forme d'ellipsoïde de révolution.

6. Corpuscules renfermant des produits pharmaceutiques selon l'une des revendications 1-5, caractérisés en ce qu'il s'agit pour le produit pharmaceutique de substances à action antibactérienne.

7. Corpuscules renfermant des produits pharmaceutiques selon la revendication 6, caractérisés en ce qu'il s'agit pour le médicament de substances du groupe des antibiotiques.

8. Corpuscules renfermant des produits pharmaceutiques selon l'une des revendications 1-5, caractérisés en ce qu'il s'agit pour le produit pharmaceutique de substances à action antivirale.

9. Corpuscules renfermant des produits pharmaceutiques selon l'une des revendications 1-5, caractérisés en ce qu'il s'agit pour le produit pharmaceutique de substances qui sont actives contre les tumeurs des tissus.

10. Corpuscules renfermant des produits pharmaceutiques selon l'une des revendications 1-9, caractérisés en ce que les produits pharmaceutiques sont utilisés en combinaisons avec des actions différentes.

**Claims**

1. Corpuscles containing medicaments and consisting of a biologically inert plastic, with one or various medicaments being present in the plastic in homogeneous distribution and capable of release, and the corpuscles being connected together, characterized in that the corpuscles have a shape which differs from the purely spherical shape, threads or wires are additionally incorporated in the corpuscles and have a tapering shape at least at one end. (sic).

2. Corpuscles containing medicaments according to Claim 1, characterized in that the threads or wires which are incorporated in the corpuscles are provided with additional holding means.

3. Corpuscles containing medicaments according to Claim 1 or 2, characterized in that the corpuscles are designed to be polygonal.

4. Corpuscles containing medicaments according to one of Claims 1–3, characterized in that the corpuscles have a polygonal or rounded cross-section which serves for firm anchoring, which can, however, be released again, in an osteosynthesis aid or in an implant.

5. Corpuscles containing medicaments according to one of Claims 1–4, characterized in that the corpuscles are designed as ellipsoid of revolution.

6. Corpuscles containing medicaments according to one of Claims 1–5, characterized in that the medicament takes the form of substances having antibacterial activity.

7. Corpuscles containing medicaments according to Claim 6, characterized in that the medicament takes the form of substances from the group of antibiotics.

8. Corpuscles containing medicaments according to one of Claims 1–5, characterized in that the medicament takes the form of substances having antiviral activity.

9. Corpuscles containing medicaments according to one of Claims 1–5, characterized in that the medicament takes the form of substances which are active against tissue tumours.

10. Corpuscles containing medicaments according to one of Claims 1–9, characterized in that medicaments with different actions are used in combination.

1/1

Fig. 1

Fig. 2

Fig. 3